# DEMANDE DE BREVET EUROPEEN

(11) **EP 1 512 452 A1**
(43) Date de publication de la demande: **09.03.2005**
(21) Numéro de dépôt: 04292133.8
(22) Date de dépôt: 03.09.2004
(51) Int. Cl.: B01D 39/16, A61M 16/10, B01D 35/30, B01D 46/24, B01D 35/14

(54) **Système de filtration pour dispositif d'aspiration par le vide**

(30) Priorité: 03.09.2003 FR 0310442
(71) Demandeur: GCE SAS, 58404 La Charité sur Loire (FR)
(72) Inventeur: Lamouret, Patrick, 77100 Meaux (FR)
(74) Mandataire: Debay, Yves

(57) **Abrégé**

La présente invention concerne un système de filtration d'un dispositif d'aspiration de vide protégeant un réseau de vide par interposition entre un bocal de recueil et le réseau de vide, caractérisé en ce qu'il comprend un couvercle (1) monté et fixé par un moyen de fixation (220) au-dessus d'un réceptacle (2), ledit réceptacle (2) accueillant dans un volume ouvert un premier filtre anti-poussière (4) et dans un volume fermé un deuxième filtre hydrophobe (3), le système assemblé comprend deux orifices dont une entrée (20) sur la surface inférieure du réceptacle mettant en communication le volume fermé vers le bocal de recueil et une sortie (10) sur la surface supérieure du couvercle (1) étant reliée au réseau de vide, les éléments infectieux passant par l'entrée (10) et étant totalement filtrés par les filtres, le filtre hydrophobe comportant des moyens d'obturation agissant dès que du liquide est mélangé aux éléments à aspirer.

## Description

La présente invention concerne un système de filtration pour dispositif d'aspiration par le vide généralement utilisé dans le milieu médical pour protéger un réseau de vide de particules infectieuses.

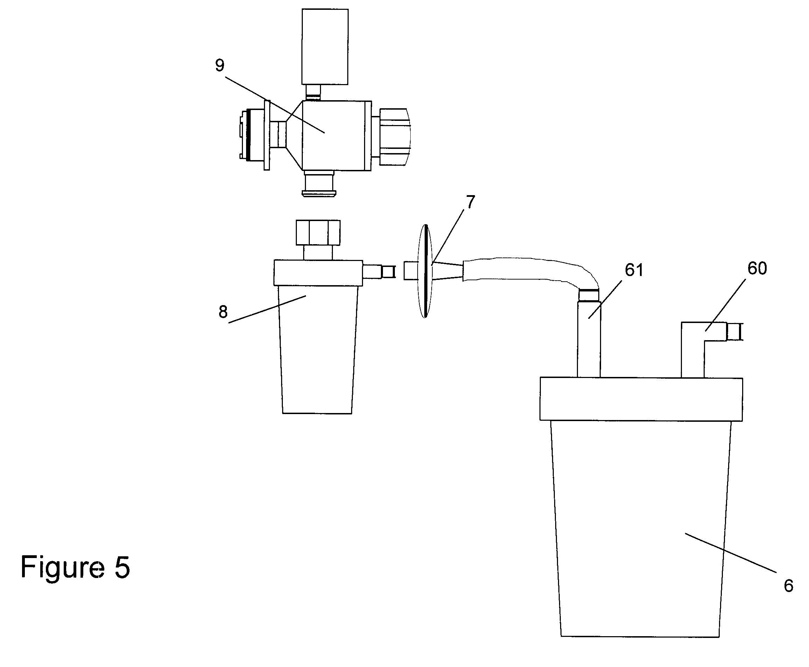

Il est connu dans l'art antérieur un dispositif de filtration pour dispositif d'aspiration du vide, présenté sur la figure 5. Il existe toute une gamme de matériel médical appelé matériel de vide permettant d'aspirer tous les liquides ou mucosités provenant d'un corps malade ou opéré. Le dispositif d'aspiration représenté sur la figure 5 comprend un bocal de recueil (6), un filtre microbien (7), un bocal de sécurité (8) et un régulateur d'aspiration de vide (9). Le bocal de recueil (6) comprend deux orifices. Un tuyau branché (60) au premier orifice est destiné à être relié au malade, un deuxième tuyau (61) branché au deuxième orifice est relié à l'entrée du filtre microbien (7). La sortie du filtre microbien (7) est reliée à l'entrée du bocal de sécurité (8). La sortie du bocal de sécurité (8) est reliée à l'entrée d'un régulateur de vide (9), dont la sortie est directement branchée au réseau de vide de l'hôpital. Lors par exemple d'une opération, un patient est branché en permanence sur ce dispositif, qui permet d'aspirer les mucosités qui peuvent ne pas être immunisées. Grâce au régulateur de vide (9), un agent médical peut régler la pression du vide, par exemple, entre -0 et -1000 millibars. Le bocal de recueil (6) se remplit de liquide. Lorsque le niveau du liquide arrive en haut du bocal de recueil (6), une soupape de trop plein (non représentée) disposée dans le bocal de recueil empêche normalement un débordement des éléments polluants, dans le cas contraire, le réseau de vide de l'hôpital peut être pollué. En effet, il peut s'avérer que la soupape de trop plein soit défaillante et laisse passer les éléments contaminateurs. Pour pallier ce problème un système de filtration, comportant un filtre microbien (7) et un bocal de sécurité (8), est placé entre le bocal de recueil (6) et le régulateur de vide (9). Ainsi, si le bocal de recueil (6) déborde, le bocal de sécurité (8) permet de jouer le rôle de tampon. Une deuxième soupape (non représentée) est disposée dans le bocal de sécurité (8), empêchant les humeurs de polluer le circuit de vide. Ce système n'est pas très efficace dû au filtre microbien (7), qui n'est pas suffisamment fin et n'empêche pas les particules en suspension de passer dans le réseau de vide. Un autre inconvénient de ce système de filtration est son coût. Le bocal de sécurité est fabriqué en matière plastique coûteux car étant prévu pour passer en autoclave à un fort degré de température afin de détruire les germes provenant d'un patient opéré et ayant pollué le bocal.

La présente invention a pour but de pallier certains inconvénients de l'art antérieur en proposant un système de filtration peu coûteux et efficace contre toute pollution d'un réseau de vide par des particules infectieuses.
Ce but est atteint par un système de filtration d'un dispositif d'aspiration de vide destiné à protéger un réseau de vide par interposition entre un bocal de recueil et le réseau de vide, le système de filtration comprenant deux orifices dont une entrée mettant en communication le système de filtration vers le bocal de recueil et une sortie pouvant être reliée au réseau de vide, les éléments à aspirer passant par l'entrée du système de filtration et étant totalement filtrés, le système de filtration étant caractérisé en ce qu'il comprend un couvercle monté et fixé par un moyen de fixation au-dessus d'un réceptacle, ledit réceptacle accueillant dans un volume ouvert un premier filtre anti-poussière et dans un volume fermé un deuxième filtre hydrophobe, la surface inférieure du réceptacle comprenant l'entrée du système de filtration, la surface supérieure du couvercle comprenant la sortie du système de filtration, le filtre anti-poussière et le filtre hydrophobe assemblés ensemble par un moyen de fixation amovible pour filtrer tous les éléments aspirés, ledit filtre hydrophobe comportant des moyens d'obturation agissant dès que du liquide est mélangé aux éléments à aspirer.
Selon une autre particularité, le système de filtration est assemblé par un système d'emboîtement des éléments comprenant différents moyens de fixation.

Selon une autre particularité, le filtre hydrophobe est réalisé en plastique poreux imprégné d'une substance devenant gélatineuse et se dilatant au contact d'une matière humide pour boucher les pores de la matière plastique, bloquant ainsi tout passage de substance au travers du plastique poreux.
Selon une autre particularité, le plastique poreux est du polyéthylène fritté, dont les pores sont imprégnés de cellulose.
Selon une autre particularité, le filtre anti-poussière est maintenu par une cloison s'appuyant sur un épaulement périphérique du réceptacle, le filtre hydrophobe venant entourer une cheminée communiquant après une ouverture dans la cloison avec le filtre anti-poussière.
Selon une autre particularité, un moyen de fixation du filtre hydrophobe sur le filtre anti-poussière est réalisé par serrage sur la cheminée.
Selon une autre particularité, le système de filtration comprend un système de blocage du filtre hydrophobe dans le réceptacle une fois le système de filtration totalement assemblé.
Selon une autre particularité, le système de maintien du filtre hydrophobe comprend au moins trois rainures séparées les unes des autres de manière homogène, disposées au fond du récipient, chacune des rainures étant située entre le bord intérieur du réceptacle et le bord extérieur de l'orifice d'entrée, lesdites rainures ayant une hauteur adaptée à la hauteur du filtre hydrophobe, permettant de bloquer celui-ci entre les rainures et la cloison du filtre anti-poussière, une fois le système de filtration assemblé.
Selon une autre particularité, la sortie du système de filtration est disposée au milieu de la surface supérieure du couvercle comprenant un orifice situé au milieu de la surface supérieure du couvercle, ledit orifice étant prolongé d'un raccord disposé en saillie par rapport au couvercle.
Selon une autre particularité, le système de filtration est de forme sensiblement cylindrique.

Selon une autre particularité, le moyen de fixation du couvercle sur le réceptacle est un système de filetage permettant ainsi de visser le couvercle sur le réceptacle.

D'autres particularités et avantages de la présente invention apparaîtront plus clairement à la lecture de la description ci-après, faite en référence aux dessins annexés, dans lesquels :
- la figure 1 représente une vue éclatée du système de filtration selon la présente invention ;
- la figure 2 représente une vue de dessus du réceptacle de la présente invention permettant de visualiser l'intérieur du réceptacle ;
- la figure 3 représente une vue éclatée du filtre anti-poussière et du filtre hydrophobe selon la présente invention ;
- la figure 4 représente une vue d'une variante de réalisation de la présente invention ;
- la figure 5 représente un schéma d'un dispositif d'aspiration par le vide connu, comprenant notamment un système de filtration de vide.

La présente invention propose un système de filtration de dispositif à aspiration par le vide. En référence à la figure 5 présentée précédemment et montrant un dispositif connu d'aspiration par le vide dans un milieu médical, le système de filtration de la présente invention remplace le bocal de sécurité et le filtre microbien disposés entre le bocal de recueil et le régulateur de dépression.

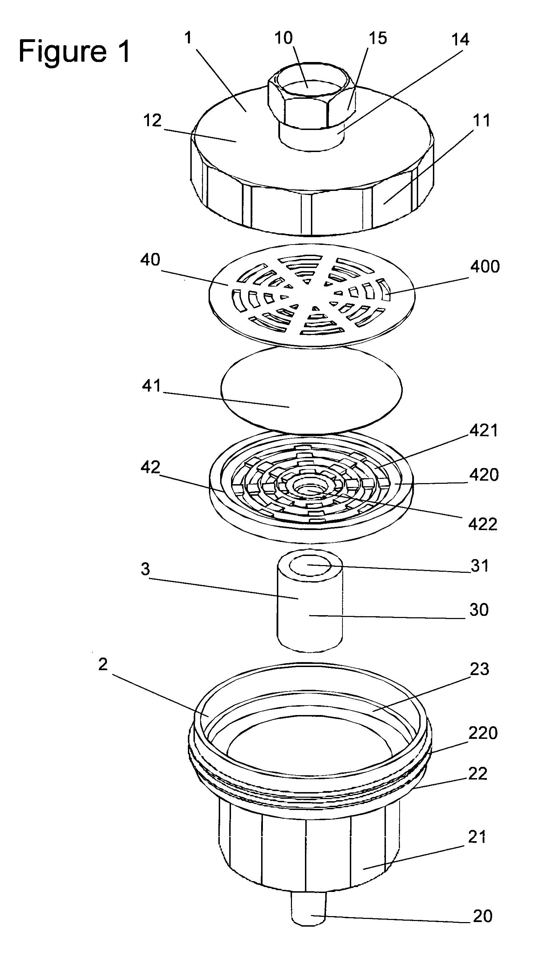

La présente invention va être décrite en référence aux figures 1, 2, 3 et 4, selon un mode de réalisation préféré de la présente invention.

La figure 1 représente un schéma du système de filtration de la présente invention, comprenant un couvercle (1) monté au-dessus d'un réceptacle (2). Une fois assemblé, le système comprend deux orifices (10 - 20). Le premier orifice (20), considéré comme l'entrée du système, est destiné à se brancher sur le bocal de recueil. Ce premier orifice (20) sera appelé orifice d'entrée du système dans la suite de la description. Le deuxième orifice (10), considéré, comme la sortie du système, se branche directement sur le régulateur d'aspiration par le vide. Ce deuxième orifice (10) sera appelé orifice de sortie du système dans le reste de la description.

La figure 1 représente une vue éclatée de la composition du système de filtration suivant un mode de réalisation de la présente invention. Le système de filtration de la présente invention comprend un réceptacle (2) permettant d'accueillir un filtre hydrophobe (3) et un filtre anti-poussière (4). Une fois les filtres placés et emboîtés dans le réceptacle (2), le couvercle est fixé par un moyen de fixation (220), sur le réceptacle (2) permettant de maintenir les filtres en son enceinte. Le système de filtration selon un mode préféré de la présente invention est de forme cylindrique.

Le fonctionnement de ce système consiste à aspirer par le vide des particules infectieuses venant d'un corps malade. Lors, par exemple d'une opération, un dispositif d'aspiration par le vide est branché sur un patient. Ce dispositif comprend un régulateur de vide branché directement sur le réseau de vide, par exemple, d'un hôpital. Ce régulateur est branché directement en entrée de manière étanche sur la sortie du système de filtration de la présente invention. L'entrée du système de filtration est branchée de manière étanche sur la sortie du bocal de recueil, par l'intermédiaire d'un tuyau pouvant être flexible. Lors de l'opération, des particules infectieuses d'un patient sont aspirées et stockées dans le bocal de recueil. Si par défaut de manipulation ou par oubli de contrôle, les particules débordent du bocal de recueil, elles sont filtrées complètement par le système de filtration de la présente invention, permettant ainsi de ne pas polluer le réseau de vide. Le trop plein de mucosités arrive dans le système de filtration, qui réagit à l'humidité des mucosités et qui bloque par colmatage l'aspiration empêchant toute remontée de matière dans le réseau de vide de l'hôpital.

En référence à la figure 1, le couvercle (1) est constitué d'un volume (11) comprenant une première ouverture (non représentée) de grande dimension disposée sur la partie inférieure du volume et une deuxième ouverture (10) plus étroite disposée sur la partie supérieure du volume. La première ouverture est de dimension sensiblement égale à l'ouverture supérieure du réceptacle (2) permettant ainsi de raccorder le couvercle (1) au réceptacle (2). Le volume (11) du couvercle est représenté par une première pièce creuse (11) de forme cylindrique pouvant être de configuration hexagonale sur la face extérieure du cylindre (11). Un disque (12) de diamètre sensiblement identique au diamètre de la pièce cylindrique est disposé sur l'extrémité supérieure de cette pièce (11) et est solidaire à cette pièce. La deuxième ouverture (10) du volume est représentée par un orifice (non présenté sur la figure), de forme circulaire, situé au milieu du disque (12), surmonté d'une cheminée (14).Le diamètre de l'orifice est de faible dimension est son centre est traversé par l'axe de la première pièce cylindrique (11). La cheminée est représentée par une deuxième pièce (14) de forme cylindrique, creuse, disposée en saillie par rapport à la première pièce cylindrique (11). Cette cheminée est solidaire du disque (12) par son extrémité inférieure et est disposée sur le disque (12) parallèlement à l'orifice (non représenté). Le diamètre intérieur de cette pièce (14) est identique au diamètre de l'orifice, l'axe de cette pièce (14) passant par le milieu de l'orifice et étant en continuité avec l'axe de la première pièce cylindrique (11). Un moyen de préhension (15) du système de filtration, pouvant être de forme hexagonale, est monté sur la bordure extérieure de l'extrémité supérieure de la cheminée (14). L'orifice du disque (12) et la cheminée (14) forment la sortie (10) du système de filtration, selon un mode de réalisation de la présente invention. Cette sortie est fixée par un système d'encliquetage connu, sur le régulateur de vide du système d'aspiration du vide.

La figure 1 présente également la composition du filtre anti-poussière (4). Ce filtre comprend trois pièces de forme circulaire ou cylindrique. Le diamètre maximum du filtre (4) a une dimension spécifique permettant d'emboîter le filtre anti-poussière (4) dans le réceptacle (1). La pièce principale de ce filtre est un disque fibreux (41) permettant d'arrêter les particules aspirées. Ce disque (41) est disposé dans une enceinte (42) de forme cylindrique, de faible hauteur, dont le diamètre intérieur est sensiblement égal au diamètre du disque fibreux (41). Cette enceinte (42) comprend un fond circulaire (420) sur lequel est disposé un système d'aération (421) connu pouvant être représenté par des ouvertures. Un orifice circulaire (422) est disposé au milieu du fond circulaire (420), l'axe de l'enceinte cylindrique (42) passant par le milieu de l'orifice circulaire (420). Le disque fibreux (41) est disposé dans le fond de l'enceinte cylindrique (422). Un couvercle de forme circulaire (40), dont la dimension du diamètre extérieur est sensiblement égale à la dimension du diamètre du disque fibreux (41), recouvre le disque fibreux (41) et s'emboîte dans l'enceinte cylindrique (42). Le couvercle comprend également un système d'aération (400) connu pouvant être représenté par des ouvertures. Une lamelle (401) du couvercle (40) du filtre anti-poussière (4) solidaire est disposée au-dessous de ce couvercle (40). La lamelle (401) est de forme circulaire, et son diamètre extérieur est inférieur au diamètre extérieur du couvercle (40). Le milieu de la lamelle (401) est situé dans l'axe du milieu du couvercle (40). Cette lamelle (401) permet de maintenir le disque fibreux (41) par appui, une fois que le filtre anti-poussière (4) est assemblé. Pour éviter plusieurs utilisations du même filtre fibreux (41) pour plusieurs patients, le couvercle (40) est fixé de manière définitive à l'enceinte cylindrique (42) par un moyen de fixation pouvant être un collage spécifique ou une soudure par ultrason.

Une cheminée (423), disposée en dessous de l'enceinte cylindrique (42) parallèlement à l'orifice circulaire (422) est solidaire de l'enceinte (42). Cette cheminée est représentée par une pièce en saillie (423) être de forme conique dont l'axe passe par le milieu de l'orifice circulaire (422) et est en continuité avec l'axe de l'enceinte cylindrique (42). Le diamètre intérieur de l'extrémité supérieure de cette pièce conique (423) est sensiblement égal au diamètre de l'orifice circulaire (422) de l'enceinte (42). Les diamètres intérieur et extérieur de l'extrémité de la pièce conique (423) sont plus petits que le diamètre de l'orifice circulaire (422) de l'enceinte (42), permettant ainsi cette forme conique.

Le filtre hydrophobe (3), représenté sur la figure 1, est constitué d'un volume (30) creux ouvert sur sa partie supérieure. Ce volume (30) peut être de forme cylindrique et son creux ouvert représenté par un logement (31) peut également être de forme cylindrique. Le volume cylindrique (30) est réalisé en polyéthylène fritté dont les pores sont imprégnés de cellulose. En présence d'un milieu humide, la cellulose gonfle et les pores du polyéthylène se colmatent bloquant ainsi l'aspiration par le vide. Le filtre hydrophobe (3) joue à la fois le rôle de filtre et de soupape de trop plein, puisqu'une fois humide la substance contenue dans ces pores gonfle et réalise un colmatage complet rendant le filtre étanche. Ce système de filtration est plus efficace que le système de clapet connu. En effet il ne laisse passer aucune éclaboussure avant de se fermer. Le filtre hydrophobe (3) est fixé de manière non définitive au filtre anti-poussière (4) par l'intermédiaire d'un moyen de fixation. Ce moyen de fixation, selon un mode de réalisation de la présente invention, peut être réalisé par serrage entre les deux filtres, en introduisant dans le logement du filtre hydrophobe la cheminée conique du filtre anti-poussière. Le logement cylindrique (31) du filtre hydrophobe a un diamètre de dimension sensiblement équivalente au diamètre de l'extrémité supérieure de la cheminée (423). Ainsi grâce à cette forme conique, le début de la cheminée (423) du filtre anti-poussière (4) est introduit facilement dans le logement (31) creux et cylindrique du filtre hydrophobe. Dès que les deux pièces sont en contact, on force l'insertion par un serrage pour maintenir les deux pièces l'une à l'autre.

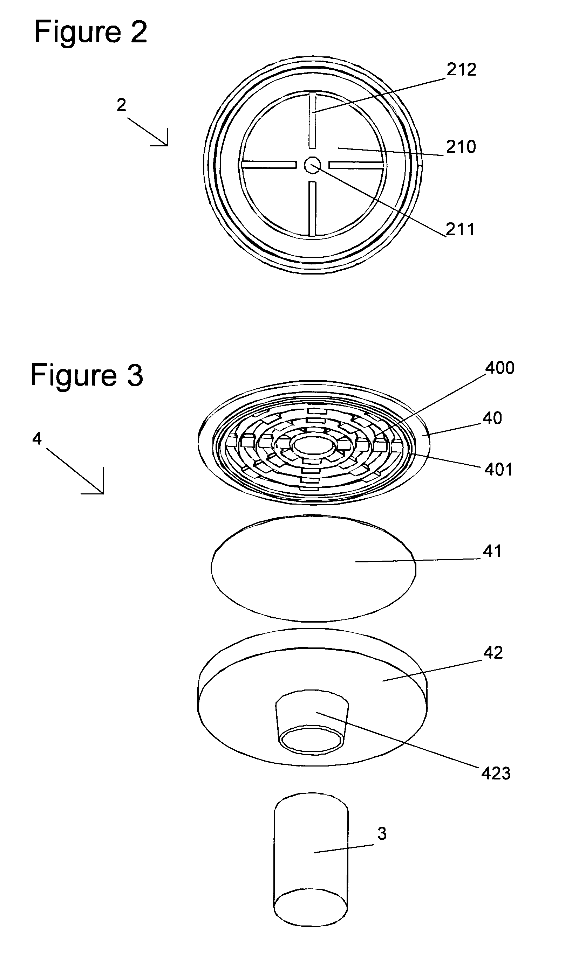

Le réceptacle (2) est présenté sur les figures 1 et 2. Il est constitué d'un volume fermé (21) disposé sur la partie inférieure du réceptacle (2) surmonté d'un volume ouvert (22) disposé sur la partie supérieure du réceptacle (2). Le volume fermé (21) comprend une première pièce de forme cylindrique pouvant être de configuration hexagonale en son extérieur, ladite pièce ayant une première ouverture de grande dimension sur son extrémité supérieure et une deuxième ouverture de dimension plus petite sur son extrémité inférieure. La deuxième ouverture est représentée par un fond circulaire au milieu duquel est agencé un orifice de faible diamètre. La hauteur de cette pièce (21) à une dimension adaptée à recevoir le filtre hydrophobe (3) emboîté dans le filtre anti-poussière et un espace libre permettant d'accueillir le trop plein de mucosités venant d'un patient malade. L'extrémité supérieure, du volume fermé (21) du réceptacle, est prolongée par le volume ouvert (22), pouvant également être de forme cylindrique. Ce volume ouvert est solidaire du volume fermé (21). La forme cylindrique du volume ouvert (22) a un diamètre intérieur plus grand que le diamètre de la première pièce cylindrique du volume fermé (21), formant ainsi un épaulement (23) entre les deux pièces. Cet épaulement (23) permet d'accueillir par emboîtement et de maintenir le filtre anti-poussière (4). La dimension du volume ouvert (22) cylindrique est de dimension sensiblement identique au diamètre du volume ouvert (11) du couvercle, permettant d'adapter le réceptacle (2) au couvercle (1) du système.

Un moyen de fixation (20) permet de maintenir un tuyau en matière flexible sur l'entrée (20) du système, permettant de relier le système de filtration à un bocal de recueil par exemple. Ce moyen de fixation (20) est représenté dans le mode de réalisation de la présente invention par une cheminée (20) pouvant être de forme conique, montée de manière solidaire sur le fond (210) du réceptacle (2) et parallèlement à l'orifice (211) situé au milieu du fond (210) du réceptacle (2). L'extrémité supérieure du cône de la cheminée (20) a un diamètre intérieur identique au diamètre de l'orifice (211). L'extrémité inférieure du cône (20) a son diamètre intérieur plus petit que le diamètre de l'extrémité supérieure du cône (20). L'axe du cône (20) passe par le milieu de l'orifice (211) sur le fond (210) du réceptacle. Le cône (20) facilite l'insertion d'un tuyau. La cheminée (20) solidaire du fond (210) du réceptacle (2) et l'orifice (210) situé au fond (210) du réceptacle (2) forment l'entrée du système de filtration. L'aspect conique de l'entrée (20) du système de filtration représente un mode de réalisation non limitatif de la présente invention.

Un moyen de maintien est disposé au fond du volume fermé du réceptacle, pour éviter que le filtre hydrophobe (3) se desserre du filtre anti-poussière (4) une fois que le système de filtration est assemblé. Dans un mode de réalisation de la présente invention, ce moyen de maintien comprend au moins trois rainures (212) disposées dans le fond (210) du réceptacle (2), avec un espacement angulaire homogène pouvant être de 120°. Dans l'exemple présenté sur la figure 2, le système de maintien comprend quatre rainures (212) diamétralement opposées les unes des autres. Chaque rainure (212) comprend deux extrémités. Une des extrémités est solidaire de la surface intérieure du volume fermé (21) du réceptacle (2). L'autre extrémité est dirigée vers l'orifice (211) situé au fond du réceptacle (210). L'espace entre les extrémités libres des rainures forme une surface ayant une dimension inférieure à la dimension de la surface située sur l'extrémité inférieure du filtre hydrophobe. Ainsi lorsque les filtres sont insérés dans le réceptacle (2), la surface inférieure du filtre hydrophobe (3) est en appui, sur les rainures (212). Lorsque le système de filtration est totalement assemblé, le filtre hydrophobe (3) est bloqué entre les rainures (212) et le filtre anti-poussière (4), ledit filtre hydrophobe (3) ne pouvant bouger.

Le couvercle (1) est fixé au réceptacle (2) par un moyen de fixation (220) pouvant être réalisé par un système de serrage par filetage. Un premier filetage est disposé sur l'intérieur du volume ouvert (11) du couvercle (1). Un deuxième filetage (220), complémentaire au premier filetage, est disposé sur l'extérieur du volume fermé (21) du réceptacle (2).

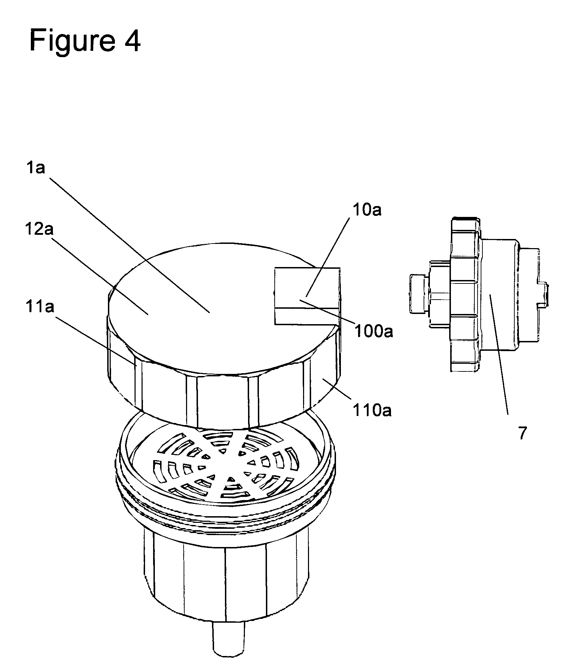

La figure 4 représente une variante de mode de réalisation de la présente invention, concernant une modification apportée sur le couvercle (1a) du système de filtration. Cette variante de réalisation permet de raccorder directement le système de filtration sur le réseau de vide par l'intermédiaire d'une prise murale (5), dans le cas d'une manipulation sans régulateur d'aspiration de vide. Dans ce mode de réalisation le couvercle (1a) comprend un volume ouvert représenté par une pièce de forme cylindrique (11 a), dont la surface extérieure est de configuration hexagonale, surmontée d'un disque solidaire (12a) de la pièce cylindrique (11a). La sortie du système de filtration (10a) est située perpendiculairement à l'axe du couvercle (1a) sur la face hexagonale (11 a) de la première pièce cylindrique. Cette sortie (10a) comprend un volume rectangulaire (100a) creux ouvert sur une extrémité, ledit volume étant disposé en saillie par rapport au disque du couvercle. L'extrémité ouverte du volume rectangulaire est située sur la périphérie du disque (12a). Cette extrémité plane est en continuité avec une surface plane (110a) de la partie extérieure hexagonale de la première pièce cylindrique (11 a) du couvercle (1a), le tout formant une unité plane. La taille du volume rectangulaire creux (100a) a une dimension adaptée permettant à une prise murale (5) d'y être introduite, tout en laissant un espace libre pour le passage du vide.

Il doit être évident pour les personnes versées dans l'art que la présente invention permet des modes de réalisation sous de nombreuses autres formes spécifiques sans l'éloigner du domaine d'application de l'invention comme revendiqué. Par conséquent, les présents modes de réalisation doivent être considérés à titre d'illustration, mais peuvent être modifiés dans le domaine défini par la portée des revendications jointes, et l'invention ne doit pas être limitée aux détails donnés ci-dessus.

## Revendications

1. Système de filtration d'un dispositif d'aspiration de vide destiné à protéger un réseau de vide par interposition entre un bocal de recueil et le réseau de vide, le système de filtration comprenant deux orifices (10-20) dont une entrée (20) mettant en communication le système de filtration vers le bocal de recueil et une sortie (10) pouvant être reliée au réseau de vide, les éléments à aspirer passant par l'entrée (10) du système de filtration et étant totalement filtrés, le système de filtration étant **caractérisé en ce qu'**il comprend un couvercle (1) monté et fixé par un moyen de fixation (220) au-dessus d'un réceptacle (2), ledit réceptacle (2) accueillant dans un volume ouvert un premier filtre anti-poussière (4) et dans un volume fermé un deuxième filtre hydrophobe (3), la surface inférieure du réceptacle (2) comprenant l'entrée (20) du système de filtration, la surface supérieure du couvercle (1) comprenant la sortie (10) du système de filtration, le filtre anti-poussière (4) et le filtre hydrophobe (3) assemblés ensemble par un moyen de fixation amovible (423) pour filtrer tous les éléments aspirés, ledit filtre hydrophobe comportant des moyens d'obturation agissant dès que du liquide est mélangé aux éléments à aspirer.

2. Système de filtration selon la revendication 1, **caractérisé en ce que** le système de filtration est assemblé par un système d'emboîtement des éléments comprenant différents moyens de fixation.

3. Système de filtration selon la revendication 1 ou 2, **caractérisé en ce que** le filtre hydrophobe (3) est réalisé en plastique poreux imprégné d'une substance devenant gélatineuse et se dilatant au contact d'une matière humide pour boucher les pores de la matière plastique, bloquant ainsi tout passage de substance au travers du plastique poreux.

4. Système de filtration selon la revendication 3, **caractérisé en ce que** le plastique poreux est du polyéthylène fritté, dont les pores sont imprégnés de cellulose.

5. Système de filtration selon une des revendications 1 à 4, **caractérisé en ce que** le filtre anti-poussière (4) est maintenu par une cloison (42) s'appuyant sur un épaulement (23) périphérique du réceptacle, le filtre hydrophobe venant entourer une cheminée (423) communiquant après une ouverture dans la cloison (42) avec le filtre anti-poussière (4).

6. Système de filtration selon une des revendications 1 à 5, **caractérisé en ce qu'**un moyen de fixation du filtre hydrophobe sur le filtre anti-poussière est réalisé par serrage sur la cheminée (423).

7. Système de filtration selon une des revendications 1 à 6, **caractérisé en ce que** qu'il comprend un système de blocage du filtre hydrophobe dans le réceptacle une fois le système de filtration totalement assemblé.

8. Système de filtration selon une des revendications 1 à 7, **caractérisé en ce que** le système de maintien du filtre hydrophobe comprend au moins trois rainures (212) séparées les unes des autres de manière homogène, disposées au fond du récipient (2), chacune des rainures (212) étant située entre le bord intérieur du réceptacle (2) et le bord extérieur de l'orifice d'entrée, lesdites rainures (212) ayant une hauteur adaptée à la hauteur du filtre hydrophobe (3), permettant de bloquer celui-ci entre les rainures (212) et la cloison (42) du filtre anti-poussière (4), une fois le système de filtration assemblé.

9. Système de filtration selon une des revendications 1 à 8, **caractérisé en ce que** la sortie du système de filtration (10) est disposée au milieu de la surface supérieure (12) du couvercle (1) comprenant un orifice situé au milieu de la surface supérieure (12) du couvercle, ledit orifice étant prolongé d'un raccord (14) disposé en saillie par rapport au couvercle (1).

10. Système de filtration selon une des revendications 1 à 9, **caractérisé en ce que** le système de filtration est de forme sensiblement cylindrique.

11. Système de filtration selon une des revendications 1 à 10, **caractérisé en ce que** le moyen de fixation du couvercle (1) sur le réceptacle (2) est un système de filetage (220) permettant ainsi de visser le couvercle (1) sur le réceptacle (2).
